# EUROPEAN PATENT SPECIFICATION

(11) **EP 2 914 346 B1**
(45) Date of publication and mention of the grant of the patent: **01.11.2017**
(21) Application number: 13763234.5
(22) Date of filing: 16.09.2013
(51) Int. Cl.: A61Q 5/04, A61Q 5/06, A61Q 5/10, A61K 8/365

(54) **PROCESS FOR STRAIGHTENING AND DYEING HAIR**
VERFAHREN ZUM GLÄTTEN UND FÄRBEN DES HAARS
PROCÉDÉ POUR DÉFRISER ET TEINDRE LES CHEVEUX

(30) Priority: 05.11.2012 EP 12191330; 10.07.2013 EP 13175916
(43) Date of publication of application: 09.09.2015
(73) Proprietor: Kao Corporation, Chuo-ku Tokyo 103-8210 (JP)
(72) Inventor: ROSE, Burkhard, 64297 Darmstadt (DE); WOOD, Jonathan, 69469 Weinheim (DE)
(74) Representative: Hoffmann Eitle
(86) International application number: PCT/EP2013/069133
(87) International publication number: WO 2014/067702

(56) References cited:
- WO-A2-2011/104282
- WO-A2-2011/139433
- WO-A2-2012/010351
- "Glyoxylic acid", Wikipedia, 8 May 2013 (2013-05-08), XP055085717, Retrieved from the Internet: URL:http://en.wikipedia.org/wiki/Glyoxylic _acid [retrieved on 2013-10-29]

## Description

### Field of the invention

The present invention relates to a method for semipermanent straightening and dyeing of the hair in two steps.

### Background of the Invention

A known method for straightening curly or frizzy hair involves the use of straightening irons. The high temperature of the iron leads to a breakage of hydrogen bonds in the keratin of the hair, achieving a temporary straightening. The hydrogen bonds are formed again by the action of moisture, so that the hair reverts back to its original shape over the time because of air humidity, and the straightening effect vanishes after washing the hair.

The shape of the hair is largely determined by the disulfide bonds linking two cysteine moieties of the hair keratin. In order to achieve a more permanent shaping of the hair, known methods involve the cleavage of the disulfide bonds by the action of a sulfide- or thio group containing reducing agent. After the hair has been brought into the desired shape, new disulfide bonds are formed by applying an oxidizing agent such as hydrogen peroxide, thus fixing the shape of the hair. The use of such agents, however, may cause damage to the hair.

As an example for this kind of hair shaping treatment, reference is made to GB 1 416 564, describing reducing compositions comprising thioglycolates or thiolactates as reducing agents and fixing compositions comprising hydrogen peroxide as an oxidizing agent. The reducing compositions may further comprise a salt of an acid such as glyoxylic acid as a buffering agent.

As an alternative to the above-described two-step reduction and oxidation process, the disulfide bridges can be cleaved by the action of an alkaline agent such as sodium hydroxide at a pH of about 11 or higher. Under these conditions, the disulfide (or cystin) moiety can undergo a disproportionation reaction under the elimination of sulfur, and is cleaved into an alpha-beta-unsaturated dehydro-alanine moiety and a cysteine moiety. After the hair has been brought into the desired shape, the dehydro-alanin moieties and the cysteine moieties form thioether bonds and combine to lanthionine, stabilizing the straightened state of the hair. Since the disulfide or cysteine moieties are converted into lanthionine moieties, this type of hair straightening process using an alkaline agent is also called lanthionization.

Both the two-stage reduction/oxidation method and the lanthionization method rely on a cleavage of the disulfide bonds and the formation of new bonds among the hair proteins, leading to an irreversible change of the shape of the hair. This means that these processes can achieve a permanent straightening, wherein the treated portion of the hair maintains its shape, and the straightening effect only vanishes because of the growth of the hair.

Recently, it has been found that carboxylic acids having a carbonyl group adjacent to the carboxy group, such as glyoxylic acid, which are known as a buffering agent in cosmetic compositions, may have a semi-permanent straightening effect when used in combination with mechanical straightening means. In this connection, semi-permanent means that the hair maintains its straightened appearance for several washing cycles. This is in contrast to permanent straightening, wherein the shape of the hair is modified irreversibly, as in the above-described methods based on the cleavage of the disulfide bonds, and in contrast to the transient straightening achieved with a straightening iron, which vanishes by the action of moisture.

In this respect, WO 2011/104282 describes a process for semi-permanent hair straightening, which involves applying a composition comprising an α-keto acid onto the hair, leaving the composition in contact with the hair for 15 to 120 minutes, drying the hair and straightening the hair with a straightening iron at a temperature of 200±50°C.

Furthermore, WO 2012/010351 describes a treatment for semi-permanent straightening of curly, frizzy or wavy hair by applying a solution of glyoxylic acid in combination with mechanical straightening, using a straightening iron at a temperature of 200±30°C. After the treatment, the hair is said to retain its shape for at least six consecutive washings.

Hair dying methods and agents may be categorized in accordance with the type of the dye and the permanency of the colour on hair. Depending on the permanency of the colour, hair dyes are usually classified as "permanent", "demi-permanent", "semi-permanent" or "temporary". Permanent and demi-permanent dyes are typically formed by oxidation dyes, while direct dyes are used for semi-permanent or temporary colouring.

Oxidation dyes are formed from low molecular intermediates which are known as "precursors" and "couplers", respectively. These molecules are small enough to penetrate into the hair. The precursors are usually aromatic para compounds such as 1,4-diaminobenzene, and are used alone or in combination with aromatic meta compounds such as 1,3-diaminobenzene as couplers. By the action of an oxidizing agent such as hydrogen peroxide under alkaline conditions, the precursor is oxidized and reacts with the coupler, thus forming the dye molecule.

Typically, the oxidative dye composition comprises ammonia as an alkalizing agent, in order to achieve a swelling of the hair and thus enhances the penetration of the precursor and the coupler into the hair. By the action of the oxidizing agent, the intermediates are coupled inside the hair. Since the size of the thus formed dye molecule is larger than the size of the intermediates, the dye molecule stays trapped inside the hair, so that a permanent colouring can be achieved.

In case of direct dyes, the actual dye molecule is applied to the hair and adheres to the hair surface because of, for example, electrostatic interactions. In contrast to the oxidation dye intermediates, the penetration of the direct dyes into the hair is relatively poor because of the larger molecule size. As a result, direct dyes can be washed out, so that the colouring is merely temporary.

Some direct dyes, in particular nitro dyes, may adhere more firmly to the hair and may penetrate deeper into the hair surface to some extent. Colouring compositions comprising these dyes are washed out less easily and thus are termed "semi-permanent".

The combination of these straightening and hair dying methods, however, has not yet been described in the state of the art.

WO 2011/139433 describes procedures for lightening the color of hair that has been recently contacted with a relaxing or straightening composition. The described procedure involves the application of an alkaline relaxing composition, followed by the application of a lightening composition having a pH from about 2 to about 7. Optionally, the lightening composition may also comprise a dye.

### Summary of the Invention

The present invention relates to a process for straightening and dyeing the hair, which comprises the following steps performed in this order:
(a) application of a first composition (Straightening Composition) having a pH of 4 or lower and comprising glyoxylic acid and/or a hydrate thereof and/or a salt thereof onto the hair from halogen, hydroxyl, amino, C₁-C₄ alkyl and C₁-C₄ alkoxy,
(b) leaving the composition on the hair for 1 to 120 minutes,
(c) drying the hair,
(d) optionally rinsing off the hair with water and drying the hair,
(e) treating the hair with an iron having a surface temperature of 130°C to 250°C, preferably 180+/-50°C,
(f) optionally rinsing and/or shampooing and drying the hair,
(g) application of a second composition (dye composition) comprising at least one hair dye, optionally at least one alkalizing agent and optionally at least one oxidizing agent onto the hair,
(h) leaving the composition on the hair for 5 to 45 minutes, rinsing off the composition and optionally shampooing and drying the hair,
wherein steps (g) to (h) are carried out directly after steps (a) to (f).

In this connection, "directly after" means that the time interval between the completion of steps (a) to (f) and the beginning of steps (g) to (h) is not more than two or three hours, preferably not more than 1 hour, more preferably not more than 30 minutes.

The process of the invention achieves a semi-permanent straightening of the hair. Generally, hair straightening may be divided into two main categories. The first is permanent straightening, wherein hair shape is irreversibly changed either by a two-step reduction and oxidation process or alternatively by treating hair with a strong alkaline composition having pH values 11.0 or above.

The second is semi-permanent straightening wherein hair is straightened relatively reversibly and returns to its approximately original shape after a certain number of washing cycles. The present invention relates to semi-permanent straightening, and does not involve the cleavage of disulfide bonds by sulfur-based reducing agents or alkali.

In another aspect, the present invention relates to a hair treatment kit comprising a straightening composition and a hair dye composition as described above, for use in the above-described process.

The dye composition and/or the straightening composition may suitably comprise further ingredients such as surfactants and/or conditioning componentas defined below, and may suitably be in the form of a solution, emulsion, cream, paste and mousse.

### Detailed Description of the Invention

Conventionally, straightening and coulouring treatment of the hair is carried out in two separate sessions which is time consuming and uneconomical for end users. Hair colouring, especially under alkaline conditions, causes usually hair damage which leads to poor colour stability.

Besides, conventional hair straightening techniques are also known to damage the hair, e.g., by the action of the disulfide-cleaving agents and/or the agents for forming new disulfide bonds. Accordingly, the combination of conventional hair straightening directly before hair colouring was likely to cause severe hair damage, leading to a dull appearance and reduced colour stability, since the dye may be washed out through the formed cracks and fissures.

In other words, if a the dyeing treatment is performed after a conventional hair straightening treatment, the colours are washed out rapidly from hair so that dyed hair loses its attractive appearance in terms of shine and vibrancy of the colours relatively quickly.

The present invention solves this problem by providing a method which achieves a straightening and coulouring of the hair in two steps in direct succession.

For this purpose, the method of the present invention uses a first composition (straightening composition) comprising glyoxylic acid, and a second composition (dye composition) comprising at least one hair dye, optionally at least one alkalizing agent and optionally at least one oxidizing agent. The hair is treated with the said compositions successively, so that the dyeing treatment is performed directly after the straightening treatment.

With the term "directly after", it is meant that the time interval between the two treatments is not more than two or three hours, preferably not more than one hour, more preferably is not more than 30 minutes.

It has surprisingly been found out by the inventors of the present invention that straightening and dyeing processes carried out in direct succession (e.g., in the same salon visit) result in less hair damage, better colour and straightening stability in comparison to two separate sessions, so that the hair treated in accordance with the inventive process of the present invention keeps its newly achieved cosmetic properties for a longer period of time, i.e., longer lasting colour and straightening effects are obtained.

### 1. The Straightening Composition

The straightening composition comprises glyoxylic acid as the active component.

The glyoxylic acid may be comprised in the composition in its free acid form. The carbonyl group adjacent to the acid group of the acid may also be present in the hydrate form. Apart from the free acid form and the hydrate thereof, salts of the acid or the hydrate may also be used.

The hydrate may be formed when providing the composition as an aqueous solution. Glyoxylic acid (H-CO-COOH) in aqueous solution is almost quantitatively present as the hydrate (H-C(OH)₂-COOH). Besides, the hydrate may also condense to dimers.

A salt of glyoxylic acid may also be used. As examples, alkali metal salts such as the sodium or potassium salt and alkaline earth metal salts such as the magnesium salt or the calcium salt may be mentioned.

The concentration of the glyoxylic acid and/or a hydrate thereof and/or salts thereof is in the range of 0.1 to 40%, preferably 0.5 to 30%, more preferably 1 to 25% and even more preferably 2.5 to 20% by weight, based on the total weight of the straightening composition.

The pH of the straightening composition is below or equal to 4.0, preferably in the range of 0.5 to 3, more preferably 1 to 2.5, as measured directly and at ambient temperature (25°C). The pH of the compositions may be adjusted using known alkaline solutions, preferably with sodium hydroxide solution.

As discussed above, conventional permanent hair shaping/straightening techniques are based on the reorganization of the disulfide bridges and involve a cleavage of the disulfide bonds either by using a sulfur-based reducing agent or an alkali agent, followed by the shaping of the hair and the formation of new bonds (i.e., disulfide bonds formed by the action of an oxidizing agent or thioether bonds, respectively). In contrast to these permanent straightening methods, the present invention does not utilize cleavage of the disulfide bonds and fixing the bonds in the new shape. Therefore, the straightening composition of the present invention does not require the presence of sulfur-based reducing agents. However, up to 2% by weight calculated to the total of the composition sulfur based reducing agents does not disturb the straightening performance of the compositions. Therefore, the straightening composition has less than 2% by weight of sulfur-based reducing agents, and preferably is free of sulfur-based reducing agents.

The straightening composition may suitably comprise further ingredients such as surfactants and/or conditioning component as defined below, and may suitably be in the form of a solution, emulsion, cream, paste and mousse.

### 2. The Dye Composition

The dye composition used in the present invention contains at least one hair dye, optionally at least one alkalizing agent and optionally at least one oxidizing agent. The at least one hair dye generally is at least one direct dye, at least one oxidation dye or a combination thereof. This means that the dye composition may be a direct dye composition or an oxidation dye composition. For instance, the oxidation dye compositions as described in EP 2 407 149 or the compositions as described in EP 2 329 809, which comprise a combination of an oxidation dye and a direct dye may be used.

The dye composition may suitably comprise further ingredients such as surfactants and/or conditioning component as defined below, and may suitably be in the form of a solution, emulsion, cream, paste and mousse.

### Direct Dye

The dye composition may comprise a direct dye. In the present invention, there are no particular limitations as to the type of direct dye, and any direct dye suitable for hair colouring may be used.

Examples of the direct dye include an anionic dye, a nitro dye, a disperse dye, and a cationic dye and mixtures thereof.

Non-limiting examples of the cationic dyes are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87 and mixtures thereof. Particularly preferred are Basic Red 51, Basic Orange 31, Basic Yellow 87 and mixtures thereof.

Non-limiting examples of the anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as the sodium or potassium salt and mixtures thereof.

Among those, the preferred anionic dyes are Acid Red 52, Acid Violet 2, Acid Red 33, Acid Orange 4, Acid Red 27 and Acid Yellow 10 and their salts. Even more preferred anionic dyes are Acid Red 52, Acid Violet 2, Acid Red 33, Acid Orange 4 and Acid Yellow 10, and their salts and mixtures thereof.

Non-limiting examples for the nitro dye are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid and mixtures thereof.

The dye composition may suitably comprise only one direct dye or a combination of direct dyes. In this respect the direct dyes of different ionic characters may also be comprised in the same composition.

The total amount of direct dye in the composition is within the range of 0.001 to 10 %, preferably 0.01 to 7.5 %, more preferably 0.05 to 5% by weight, based on the total weight of the dye composition.

If the dye composition only comprises direct dyes, an oxidizing agent is not required for colouring the hair. In case lightening of the hair colour is desired, an oxidizing agent may be included into the composition.

### Oxidation Dye

In case the dye composition comprises an oxidation dye, it is typically of a two-component type, wherein the first component comprises the oxidation dye intermediates (precursor and coupler) and an alkalizing agent, while the second component comprises an oxidizing agent such as hydrogen peroxide. The two components are typically stored separately and combined before application onto the hair.

In the present invention, there are no particular limitations as to the oxidation dye intermediates, any known precursors and couplers normally used in a hair dye product may suitably be used.

Non-limiting examples of the precursor include para-phenylenediamine, toluene-2,5-diamine, 2-chloro-para-phenylenediamine, N-methoxyethyl-para-phenylenediamine, N,N-bis(2-hydroxyethyl)-para-phenylenediamine, 2-(2-hydroxyethyl)-para-phenylenediamine, 2,6-dimethyl-para-phenylenediamine, 4,4'-diaminodiphenylamine, 1,3-bis(N-(2-hydroxyethyl)-N-(4-aminophenyl)amino)-2-propanol, PEG-3,3,2'-para-phenylenediamine, para-aminophenol, para-methylaminophenol, 3-methyl-4-aminophenol, 2-aminomethyl-4-aminophenol, 2-(2-hydroxyethylaminomethyl)-4-aminophenol, ortho-aminophenol, 2-amino-5-methylphenol, 2-amino-6-methylphenol, 2-amino-5-acetamide phenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 4,5-diamino-1-hydroxyethylpyrazole, and salts of these substances and their mixtures.

Non-limiting examples of the coupler include meta-phenylenediamine, 2,4-diaminophenaxyethanol, 2-amino-4-(2-hydroxyethylamino)anisole, 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, meta-aminophenol, 2-methyl-5-aminophenol, 2-methyl-5-(2-hydraxyethylamino)phenol, 2,4-dichloro-3-aminophenol, 2-chloro-3-amino-6-methylphenol, 2-methyl-4-chloro-5-aminophenol, N-cyclopentyl-meta-aminophenol, 2-methyl-4-methoxy-5-(2-hydroxyethylamino)phenol, 2-methyl-4-fluoro-5-aminophenol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, and salts of these substances and their mixtures.

The concentration of the dye precursors and couplers each is in the range of 0.001 to 10%, preferably 0.01 to 7.5%, more preferably 0.1 to 5% by weight based on the total weight of the dye composition.

The oxidation dye composition further comprises an alkalizing agent. Suitable non-limiting examples of the alkalizing agent include ammonia and a salt thereof; alkanolamine such as monoethanolamine, isopropanolamine, 2-amino-2-methyl propanol, and 2-aminobutanol, and a salt thereof; alkanediamine such as 1,3-propanediamine and a salt thereof; and carbonate such as guanidine carbonate, sodium carbonate, potassium carbonate, sodium bicarbonate, and potassium bicarbonate and their mixtures. The concentration of the alkalizing agent is in the range of 0.1 to 15% preferably 0.5 to 10%, more preferably 1 to 7.5% by weight, based on the total weight of the dye composition.

In the oxidation dye composition, the component comprising the oxidizing agent is stored separately and mixed with the dye component prior to application onto hair. Suitable non-limiting examples of the oxidizing agent include hydrogen peroxide, urea peroxide, melamin peroxide and sodium bromate. Among these oxidizing agents, hydrogen peroxide is preferable.

The concentration of the at least one oxidizing agent, preferably hydrogen peroxide, is in the range of 1 to 20%, preferably 1 to 15%, more preferably 1 to 12% and even more preferably 2 to 9% by weight, based on the total weight of the dye composition.

When stored separately, the pH of the component comprising the oxidizing agent is preferably 2 to 6, more preferably 2.5 to 4. The pH may be adjusted by means of suitable buffering agents.

After mixing the dye component with the oxidizing component, the pH of the dye composition is typically in the range of 6 to 11, preferably 6.5 to 10.5 and more preferably 6.8 to 10, measured at ambient temperature (25°C).

The oxidation dye composition may additionally comprise at least one direct dye. The direct dyes mentioned above are all suitable for this purpose.

### 3. Surfactant

The dye composition and the straightening composition may comprise a surfactant. As the surfactant, any of a cationic surfactant, a nonionic surfactant, an amphoteric surfactant and an anionic surfactant can be used. It is also possible to use two or more types of surfactants in combination.

The cationic surfactant is preferably a mono-long chain alkyl quaternary ammonium salt, having a C₈-C₂₄ alkyl residue and three C₁-C₄ alkyl residues.

Preferably at least one mono alkyl quaternary ammonium surfactant is selected from the compounds with the general formula 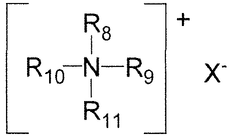 wherein R₈ is a saturated or unsaturated, branched or straight alkyl chain with 8-22 C atoms or

R₁₂-CO-NH-(CH₂)ₙ-

wherein R₁₂ is a saturated or unsaturated, branched or straight alkyl chain with 7-21 C atoms and n is an integer of 1 - 4, or

R₁₂-CO-O-(CH₂)ₙ-

wherein R₁₂ is a saturated or unsaturated, branched or straight alkyl chain with 7-21 C atoms and n is an integer of 1 - 4, and
R₉, R₁₀ and R₁₁ are independent from each other an alkyl group with 1 to 4 carbon atoms, hydroxyl alky chain with 1 to 4 carbon atoms, or ethoxy or propoxy group with a number of ethoxy or propoxy groups varying in the range of 1 to 4, and X is chloride, bromide, methosulfate or ethosulfate.

Suitable cationic surfactants are, for example, long-chain quaternary ammonium compounds which can be used alone or in admixture with one another, such as cetyl trimethyl ammonium chloride, myristyl trimethyl ammonium chloride, behentrimonium chloride, trimethyl cetyl ammonium bromide, stearyl trimethyl ammonium chloride, stearyl trimonium chloride and stearamidopropyltrimonium chloride.

Examples of the nonionic surfactant include polyoxy-C₁₋₄-alkylene C₈₋₂₄-alkyl ether, polyoxy-C₁₋₄-alkylene C₈₋₂₄-alkylene alkenyl ether, higher (C₁₂-C₂₄) fatty acid sucrose ester, polyglycerin C₈₋₂₄-fatty acid ester, higher (C₁₂-C₂₄) fatty acid mono- or diethanolamide, polyoxyethylene hardened castor oil, polyoxyethylene sorbitan C₈₋₂₄-fatty acid ester, polyoxyethylene sorbit C₈₋₂₄-fatty acid ester, C₈₋₂₄-alkyl saccharide surfactant, C₈₋₂₄-alkylamine oxide, and C₈₋₂₄-alkylamidoamine oxide.

Examples of the amphoteric surfactant include an imidazoline-based surfactant, a carbobetaine-based surfactant, an amidobetaine-based surfactant, a sulfobetaine-based surfactant, a hydroxysulfobetaine-based surfactant and an amidosulfobetaine-based surfactant.

Examples of the anionic surfactant include alkylbenzenesulfonate, alkyl or alkenyl ether sulfate, alkyl or alkenyl sulfate, olefin sulfonate, alkanesulfonate, saturated or unsaturated fatty acid salts, alkyl or alkenyl ether carboxylate, α-sulfo fatty acid salts, N-acylamino acid type surfactants, phosphoric acid mono- or diester type surfactants, and sulfosuccinate. Examples of the alkyl ether sulfate include polyoxyethylene alkyl ether sulfate. Examples of the counterion for the anionic residues of these surfactants include an alkalimetal ion such as sodium ion or potassium ion; an alkaline earth metal ion such as calcium ion or magnesium ion; an ammonium ion; and an alkanolamine having 1 to 3 alkanol groups each having 2 or 3 carbon atoms (for example, monoethanolamine, diethanolamine, triethanolamine, or triisopropanolamine).

The surfactant can be used singly or in combination of two or more kinds. When adding a surfactant to the dye composition and/or the straightening composition, the content thereof usually is 0.05 to 10% wt.%, more preferably 0.1 to 5 wt.%, based on the total weight of the dye composition and the straightening composition, respectively.

### 4. Conditioning Component

The dye composition and the straightening composition may optionally comprise a conditioning component suitable for application to the hair. The conditioning component is an oil or polymer which adheres to the hair and improves the feel and the manageability.

When using the conditioning component, the total amount thereof is preferably 0.01 to 30 wt.%, more preferably 0.05 to 20 wt.%, and even more preferably 0.1% to 10 wt.%, based on the total weight of the dye composition and the straightening composition, respectively.

Examples of the conditioning component generally include silicones, higher alcohols, and organic conditioning oils (for example, hydrocarbon oil, polyolefin and fatty acid ester). The composition may comprise a single type of conditioning component, or two or more in combination.

### Silicones

In order to improve the feel of use, the dye composition and/or the straightening composition preferably contains a silicone. Examples of the silicone include dimethylpolysiloxane, and modified silicone (for example, amino-modified silicone, fluorine-modified silicone, alcohol-modified silicone, polyether-modified silicone, epoxy-modified silicone, or alkyl-modified silicone), but dimethylpolysiloxane, polyether-modified silicone and amino-modified silicone are preferred.

The dimethylpolysiloxane may be any cyclic or non-cyclic dimethylsiloxane polymer, and examples thereof include SH200 series, BY22-019, BY22-020, BY11-026, B22-029, BY22-034, BY22-050A, BY22-055, BY22-060, BY22-083, FZ-4188 (all by Dow Corning Toray Co., Ltd.), KF-9008, KM-900 series, MK-15H, and MK-88 (all by Shin-Etsu Chemical Co., Ltd.).

The polyether-modified silicone may be any silicone having a polyoxyalkylene group, and the group constituting the polyoxyalkylene group may be an oxyethylene group or an oxypropylene group. More specific examples include KF-6015, KF-945A, KF-6005, KF-6009, KF-6013, KF-6019, KF-6029, KF-6017, KF-6043, KF-353A, KF-354A, KF-355A (all by Shin-Etsu Chemical Co., Ltd.), FZ-2404, SS-2805, FZ-2411, FZ-2412, SH3771M, SH3772M, SH3773M, SH3775M, SH3749, SS-280X series, BY22-008 M, BY11-030, and BY25-337 (all by Dow Corning Toray Co., Ltd.).

The amino-modified silicone may be any silicone having an amino group or an ammonium group, and examples thereof include an amino-modified silicone oil having all or a part of the terminal hydroxyl groups capped with a methyl group or the like, and an amodimethicone which does not have the terminals capped. A preferred example of the amino-modified silicone may be a compound represented by the following formula: 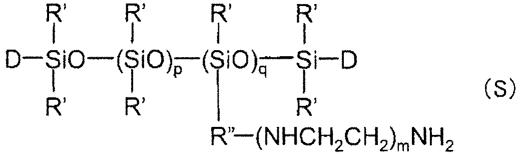 wherein R' represents a hydroxyl group, a hydrogen atom or R^{X}; R^{X} represents a substituted or unsubstituted monovalent hydrocarbon group having 1 to 20 carbon atoms; D represents R^{X}, R"- (NHCH₂CH₂)ₘNH₂, OR^{X}, or a hydroxyl group; R" represents a divalent hydrocarbon group having 1 to 8 carbon atoms; m represents a number from 0 to 3; p and q represent numbers, the sum of which is, as a number average, equal to or greater than 10 and less than 20,000, preferably equal to or greater than 20 and less than 3000, more preferably equal to or greater than 30 and less than 1000, and even more preferably equal to or greater than 40 and less than 800.

Specific examples of suitable commercially available products of the amino-modified silicone include amino-modified silicone oils such as SF8452C, SS-3551 (all by Dow Corning Toray Co., Ltd.), KF-8004, KF-867S, and KF-8015 (all by Shin-Etsu Chemical Co., Ltd.); and amodimethicone emulsions such as SM8704C, SM8904, BY22-079, FZ-4671, and FZ-4672 (all by Dow corning Toray Co., Ltd.).

The total content of these silicones in the compositions of the present invention is usually 0.1 to 20 wt.%, preferably 0.2% to 10 wt.% and more preferably 0.5 to 5 wt.%, based on the total weight of the dye composition and the straightening composition, respectively.

### Oil component

For improving the feel upon use, the dye composition and/or the straightening composition may also include an organic conditioning oil. The organic conditioning oil that is suitably used as a conditioning component is preferably a low-viscosity and water-insoluble liquid, and is selected from a hydrocarbon oil having at least 10 carbon atoms, a polyolefin, a fatty acid ester, a fatty acid amide, a polyalkylene glycol, and mixtures thereof. The viscosity of such an organic conditioning oil as measured at 40°C is preferably 1 to 200 mPa·s, more preferably 1 to 100 mPa·s, and even more preferably 2 to 50 mPa·s. For the determination of the viscosity, a capillary viscometer may be used.

Examples of the hydrocarbon oil include a cyclic hydrocarbon, a linear aliphatic hydrocarbon (saturated or unsaturated), and a branched aliphatic hydrocarbon (saturated or unsaturated), and polymers or mixtures thereof are also included. The linear hydrocarbon oil preferably has 12 to 19 carbon atoms. The branched hydrocarbon oil includes hydrocarbon polymers, and preferably has more than 19 carbon atoms.

The polyolefin is a liquid polyolefin, more preferably a liquid poly-α-olefin, and even more preferably a hydrogenated liquid poly-α-olefin. The polyolefin used herein is prepared by polymerizing an olefin monomer having 4 to 14 carbon atoms, and preferably 6 to 12 carbon atoms.

The fatty acid ester may be, for example, a fatty acid ester having at least 10 carbon atoms. Examples of such a fatty acid ester include esters having a hydrocarbon chain derived from a fatty acid and an alcohol (for example, monoesters, polyhydric alcohol esters, or di- and tricarboxylic acid esters). The hydrocarbon group of these fatty acid esters may have another compatible functional group such as an amide group or an alkoxy group as a substituent, or the hydrocarbon group may be covalently bonded to those functional groups. More specifically, an alkyl and alkenyl ester of a fatty acid having a fatty acid chain having 10 to 22 carbon atoms, a carboxylic acid ester of an aliphatic alcohol having an aliphatic chain derived from an alkyl and/or alkenyl alcohol having 10 to 22 carbon atoms, and a mixture thereof are suitably used. Specific examples of these preferred fatty acid esters include isopropyl isostearate, hexyl laurate, isohexyl laurate, isohexyl palmitate, isopropyl palmitate, decyl oleate, isodecyl oleate, hexadecyl stearate, decyl stearate, dihexadecyl adipate, lauryl lactate, myristyl lactate, cetyl lactate, oleyl stearate, oleyl oleate, oleyl myristate, lauryl acetate, cetyl propionate and dioleyl adipate.

Further suitable oil components are natural oils such as paraffin oil and natural triglycerides.

Suitable natural triglycerides are argan oil, shea butter oil, karite oil, olive oil, almond oil, avocado oil, ricinus oil, coconut oil, palm oil, sesame oil, peanut oil, sunflower oil, peach kernel oil, wheat germ oil, macadamia nut oil, macadamia oil, night primrose oil, jojoba oil, castor oil, soya oil, lanolin, passiflora oil, black cumin oil, borage oils, grapeseed oil, hempseed oil, kukui nut oil, and rosehip oil.

The organic conditioning oil may be used in combination of two or more kinds, and the total concentration is typically in the range of 0.1 to 20 wt.%, preferably 0.2 to 10 wt.%, more preferably 0.5 to 5 wt.%, based on the total weight of the dye composition and the straightening composition, respectively.

### Alcohols

From the viewpoint of improving the sense of touch and stability, the hair dye composition and/or the straightening composition may also contain a higher alcohol having 8 carbon atoms or more. Usually, the higher alcohol has 8 to 22 carbon atoms, and preferably 16 to 22 carbon atoms. Specific examples thereof include cetyl alcohol, stearyl alcohol, behenyl alcohol, and mixtures thereof.

The higher alcohol may be used in combination of two or more kinds, and the content thereof is typically 0.1 to 20 wt.%, preferably 0.2 to 10 wt.%, more preferably 0.5 to 5 wt.%, based on the total weight of the dye composition and the straightening composition, respectively.

Additionally polyols may suitably be comprised in the compositions. Examples of the polyalkylene glycol include polyethylene glycol and polypropylene glycol, and a mixture of the two may be used, or a copolymer of ethylene oxide and propylene oxide may also be used.

Besides, the dye composition may comprise further ingredients conventionally used in the field of cosmetics, such as preservatives, chelating agents, stabilizers, oxidation inhibitors, plant extracts, ultraviolet absorbers, vitamins, dyes, and fragrances.

### 5. Hair Treatment Method

The process of the present invention involves two stages, namely the straightening (steps (a) to (f)) and the colouring (steps (g) to (h)) of the hair.

The straightening stage achieves a semi-permanent straightening of the hair, utilizing glyoxylic acid as the active agent. The straightening effect is not achieved by cleaving the disulfide bonds by reduction or the action of strong alkali. Accordingly, the usage of a reducing composition or an alkaline relaxer (lanthionization agent) is not required.

In step (a), the straightening composition is applied to the hair. The application ratio of hair to composition by weight is preferably 0.5:2 to 2:0.5, more preferably 0.5:1 to 1:0.5, even more preferably about 1:1.

Subsequent to the application, the hair straightening composition is left on the hair for 1 to 120 minutes, preferably 5 to 90 minutes, more preferably 10 to 60 minutes, and in particular 15 to 45 minutes at a temperature range of 20 to 45°C and preferably at ambient temperature (step (b)). The time composition left on the hair depends on the type of the hair, whether the hair is damaged, or resistant.

After the processing, the hair is dried in order to avoid an excessive steam generation in the subsequent step of treating the hair with the iron (step (c)). Typically, a hair dryer is used for this purpose. It is preferable to dry the hair under continuous combing in order to prevent entanglement of the hair.

Optionally, the hair may be rinsed off with water and dried once again (step (d)).

Subsequent to the drying, the hair is treated with an iron (step (e)). A usual straightening iron may be used for this purpose. In order to prevent damage to the hair, the surface temperature of the iron is 250°C or lower, preferably 230°C or lower, more preferably 200°C or lower. For achieving a good straightening effect, the temperature of the iron is 130°C or higher, preferably 150°C or higher, more preferably 170° or higher. The iron may preferably have a surface temperature of 180±50°C, more preferably 170 to 200°C.

Optionally, the hair may be rinsed and/or shampooed and dried before applying the dye composition (step (f)).

The colouring steps (g) to (h) are performed directly after the straightening steps (a) to (f).

Then, the second composition (dye composition) is applied to the hair (step (g)) and left on the hair for 5 to 45 minutes, preferably 5 to 30 minutes and is rinsed off with water (step (h)). Optionally, the hair may be shampooed and dried.

The application ratio of hair to dye composition by weight is preferably 0.5:2 to 2:0.5, more preferably 0.5:1 to 1:0.5, even more preferably about 1:1.

### Examples

The following examples are to illustrate the invention but not to limit it.

### Example 1:

**Straightening Composition**

| % w/w | |
|---|---|
| 10.00 | - Glyoxylic Acid |
| 1.50 | - Amodimethicone |
| 0.30 | - Fragrance / Parfum |
| q.s. to pH 1.5 | - Sodium hydroxide |
| To 100 | - Water |

**Direct Dye Composition**

| % w/w | |
|---|---|
| 1.50 | - Amodimethicone |
| 1.40 | - Hydroxyethylcellulose |
| 0.30 | - Fragrance / Parfum |
| 0.55 | - Acid Red 52 / C.I. 45100 |
| 0.28 | - Basic red 51 |
| 0.01 | - HC red 3 |
| To 100 | - Water |

The straightening composition is applied to the pre-shampooed hair at a ratio of about 1:1, rubbed into the hair and left on the hair for about 30 minutes. Then, the hair is dried with a hair drier. A straightening iron having a temperature of about 200°C is used for the subsequent straightening.

Directly afterwards, the hair is rinsed off with water and treated with the direct dye composition.

### Example 2:

**Oxidative Hair Dye Composition for permanent colour**

| % w/w | |
|---|---|
| 0.25 | - Ammonium chloride |
| 8.00 | - Ammonia 25% |
| 0.30 | - Fragrance / Parfum |
| 0.55 | - p-toluenediamine |
| 0.28 | - resorcinol |
| 0.01 | - m-phenylenediamine |
| To 100 | - Water |
| pH: 9.5 | |

The hair is straightening in the same manner as described in Example 1. Then, the oxidative hair dye composition is mixed with a 6% H₂O₂ solution at a weight ratio of 1:1 and applied to the hair.

### Example 3:

**Demi-permanent Dye Composition**

| % w/w | |
|---|---|
| 0.25 | - Ammonium chloride |
| 0.80 | - Monoethanolamine |
| 0.30 | - Fragrance / Parfum |
| 0.55 | - p-toluenediamine |
| 0.28 | - resorcinol |
| 0.01 | - m-phenylenediamine |
| To 100 | - Water |
| pH: 6.8 | |

The hair is straightening in the same manner as described in Example 1. Then, the demi-permanent hair dye composition is mixed with a 6% H₂O₂ solution at a weight ratio of 1:1 and applied to the hair.

The hair straightened and dyed with the compositions of the above Examples 1-3 shows long lasting colour and the hair stays straight for a long period of time.

### Test Example

Four streaks (1 g each) of blonde human hair (level 9, sourced from International Hair Importers & Products, NY, USA) were used. Two of the streaks served as reference samples while the other two were subjected to the straightening treatment.

An aqueous solution containing 13.0 wt.% of glyoxylic acid monohydrate (corresponding to 10.4 wt.% glyoxylic acid) and 1.6 wt.% of a thickener (dehydoxanthan gum) and having a pH of 1.5 (adjusted with NaOH) was used as the straightening composition. The composition was applied to the hair with a comb at a ratio of 1:1 and processed for 15 minutes at a temperature of 40°C. Then, the hair was dried with a blow drier and straightened with a flat iron (temperature 230°C, 6 strokes). Afterwards, the hair was shampooed and dried again with a hair dryer.

The samples of the straightened hair and the reference samples were then subjected to the colouring treatment using oxidative hair dye compositions (Goldwell^{R} Topchic^{R} Hair Color 6NN, dark blonde and Topchic^{R} Hair Color 6RR, red, both available from Kao Germany GmbH).

Each of the oxidative hair dye compositions was mixed with 6% hydrogen peroxide solution at a weight ratio 1:1 and applied to one straightened streak and one reference streak in a weight ratio of hair to colour of 1:3. The dye compositions were evenly distributed on the streaks with a brush. Then the streaks were held at a temperature of 40°C for 30 minutes and rinsed off with water and shampooed. Afterwards the streaks were allowed to dry at room temperature for one hour, the colour was measured in terms of L*a*b values.

For evaluating the wash fastness, each streak was immersed in 100 ml of a 5% sodium lauryl ether sulfate solution (pH adjusted to approximately 5.6 with citric acid) and treated in a shaking bath for 15 minutes at a temperature of 30°C and an shaking rate of 100 min⁻¹.

After rinsing off the streaks with water and drying for one hour at ambient temperature, the colour of the streaks was again measured in terms of the L*a*b values, and the colour difference (ΔE value) was determined.

For the streaks dyed with "Topchic^{R} Hair Color 6NN" (dark blonde), the colour difference of the reference streak was ΔE = 5.89 and the colour difference of the streak straightened by the glyoxylic acid treatment was ΔE = 1.46.

For the red dyed streaks ("Topchic^{R} Hair Color 6RR"), the colour difference for the reference streak was ΔE = 7.34 while the colour difference for the streak straightened by the glyoxylic acid treatment was ΔE = 5.38.

A visual inspection of the streaks by a panel of five experienced hair stylist led to the evaluation that for both, the dark blonde and the red dyed streaks, the glyoxylic acid treated streaks have a better colour stability.

This shows that the method of the present invention does not only allow a straightening and colouring in the same treatment session, but also achieves a substantial improvement of the wash fastness and colour durability.

## Claims

1. Process for straightening and dyeing the hair, **characterized in that** it comprises the following steps in this order:
(a) application of a first composition (Straightening Composition) having a pH of 4 or lower and comprising glyoxylic acid and/or a hydrate thereof and/or a salt thereof onto the hair;
(b) leaving the composition on the hair for 1 to 120 minutes,
(c) drying the hair
(d) optionally rinsing off the hair with water and drying the hair,
(e) treating the hair with an iron having a surface temperature of 130 to 250°C, preferably 180 ± 50°C,
(f) optionally rinsing and/or shampooing and drying the hair,
(g) application of a second composition (dye composition) comprising at least one hair dye, optionally at least one alkalizing agent and optionally at least one oxidizing agent onto the hair,
(h) leaving the composition on the hair for 5 to 45 minutes, rinsing off the composition and optionally shampooing and drying the hair,
wherein steps (g) to (h) are carried out directly after steps (a) to (f).

2. The process according to claim 1, **characterized in that** the dye composition comprises one or more direct dyes, preferably selected from anionic, cationic, and nitro dyes.

3. The process according to claim 1 or 2, **characterized in that** the dye composition comprises oxidative dye precursors and couplers.

4. The process according to any of the claims 1 to 3, **characterized in that** the dye composition comprises an alkalizing agent.

5. The process according to any of the claims 1 to 4, **characterized in that** the dye composition comprises an oxidizing agent.

6. The process according to claim 5, **characterized in that** the oxidizing agent is hydrogen peroxide.

7. The process according to any of the claims 1 to 6, **characterized in that** the straightening composition comprises the glyoxylic acid and/or a hydrate thereof and/or a salt thereof at a concentration in the range of 0.1 to 40% by weight, calculated on the basis of the total weight of the straightening composition.

8. The process according to any of the claims 1 to 7, **characterized in that** the straightening composition has a pH of 0.5 to 3.

9. The process according to any of the claims 1 to 8, **characterized in that** the straightening composition and/or the dye composition further comprises one or more conditioning components.

10. The process according to claim 9, wherein the conditioning component is a silicone.

11. The process according to any of the claims 1 to 10, wherein the straightening composition comprises less than 2% of sulfur-based reducing agents.

12. The process according to any of the claims 1 to 11, **characterized in that** the straightening composition is free of any sulfur-based reducing agents.

13. The process according to any of the claims 1 to 12, **characterized in that** the straightening composition and/or the dye composition is applied onto the hair in a weight ratio of hair to composition in the range of 0.5:2 to 2:0.5.

14. The process according to any of the claims 1 to 13, **characterized in that** the temperature of the iron is in the range of 170 to 200°C.

15. The process according to any of the claims 1 to 14, wherein the combination with the application of a reducing composition or an alkaline relaxer is excluded.

16. Hair treatment kit for straightening and dyeing hair, comprising a straightening composition, which has a pH of 4 or lower and comprises glyoxylic acid and/or a hydrate thereof and/or a salt thereof; and a dye composition, which comprises at least one hair dye, optionally at least one alkalizing agent and optionally at least one oxidizing agent.

17. Use of the hair treatment kit as defined in claim 16 for straightening and dyeing the hair.

18. The use according to claim 17, wherein the straightening and dyeing is performed according to the process as defined in any of the claims 1 to 15.

## Patentansprüche

1. Verfahren zum Begradigen und Färben von Haar, **dadurch gekennzeichnet, dass** es die folgenden Schritte in dieser Reihenfolge enthält:
(a) Auftragen einer ersten Zusammensetzung (Begradigungszusammensetzung) mit einem pH von 4 oder weniger und enthaltend Glyoxylsäure und/oder ein Hydrat davon und/oder ein Salz davon, auf das Haar,
(b) Lassen der Zusammensetzung auf dem Haar für 1 bis 120 Minuten,
(c) Trocknen des Haars,
(d) wahlweise Abspülen des Haars mit Wasser und Trocknen des Haars,
(e) Behandeln des Haars mit einem Eisen mit einer Oberflächentemperatur von 130 bis 250°C, bevorzugt 180 ± 50°C,
(f) wahlweises Spülen und/oder Shampoonieren und Trocknen des Haars,
(g) Auftragen einer zweiten Zusammensetzung (Färbezusammensetzung), enthaltend zumindest einen Haarfarbstoff, wahlweise zumindest ein Alkalisiermittel und wahlweise ein Oxidationsmittel, auf das Haar,
(h) Lassen der Zusammensetzung auf dem Haar für 5 bis 45 Minuten, Abspülen der Zusammensetzung und wahlweises Shampoonieren und Trocknen des Haars,
worin die Schritte (g) bis (h) direkt nach den Schritten (a) bis (f) durchgeführt werden.

2. Verfahren gemäß Anspruch 1, **dadurch gekennzeichnet, dass** die Färbezusammensetzung einen oder mehrere direktziehende Farbstoffe enthält, bevorzug ausgewählt aus anionischen, kationischen und Nitrofarbstoffen.

3. Verfahren gemäß Anspruch 1 oder 2, **dadurch gekennzeichnet, dass** die Färbezusammensetzung oxidative Farbstoffvorläufer und -kuppler enthält.

4. Verfahren gemäß einem der Ansprüche 1 bis 3, **dadurch gekennzeichnet, dass** die Farbstoffzusammensetzung ein Alkalisiermittel enthält.

5. Verfahren gemäß einem der Ansprüche 1 bis 4, **dadurch gekennzeichnet, dass** die Färbezusammensetzung ein Oxidationsmittel enthält.

6. Verfahren gemäß Anspruch 5, **dadurch gekennzeichnet, dass** das Oxidationsmittel Wasserstoffperoxid ist.

7. Verfahren gemäß einem der Ansprüche 1 bis 6, **dadurch gekennzeichnet, dass** die Begradigungszusammensetzung die Glyoxylsäure und/oder ein Hydrat davon und/oder ein Salz davon bei einer Konzentration im Bereich von 0,1 bis 40 Gew.% enthält, berechnet auf der Basis des Gesamtgewichtes der Begradigungszusammensetzung.

8. Verfahren gemäß einem der Ansprüche 1 bis 7, **dadurch gekennzeichnet, dass** die Begradigungszusammensetzung einen pH von 0,5 bis 3 hat.

9. Verfahren gemäß einem der Ansprüche 1 bis 8, **dadurch gekennzeichnet, dass** die Begradigungszusammensetzung und/oder die Färbezusammensetzung weiterhin eine oder mehrere Konditionierkomponenten enthält.

10. Verfahren gemäß Anspruch 9, worin die Konditionierkomponente ein Silikon ist.

11. Verfahren gemäß einem der Ansprüche 1 bis 10, worin die Begradigungszusammensetzung weniger als 2 % Reduktionsmittel auf Schwefelbasis enthält.

12. Verfahren gemäß einem der Ansprüche 1 bis 11, **dadurch gekennzeichnet, dass** die Begradigungszusammensetzung frei von irgendwelchen Reduktionsmitteln auf Schwefelbasis ist.

13. Verfahren gemäß einem der Ansprüche 1 bis 12, **dadurch gekennzeichnet, dass** die Begradigungszusammensetzung und/oder die Färbezusammensetzung auf das Haar in einem Gewichtsverhältnis von Haar zur Zusammensetzung im Bereich von 0,5:2 bis 2:0,5 betragen wird.

14. Verfahren gemäß einem der Ansprüche 1 bis 13, **dadurch gekennzeichnet, dass** die Temperatur des Eisens im Bereich von 170 bis 200°C ist.

15. Verfahren gemäß einem der Ansprüche 1 bis 14, worin die Kombination mit der Auftragung einer Reduktionszusammensetzung oder eines alkalischen Relaxers ausgeschlossen ist.

16. Haarbehandlungskit zum Begradigen und Färben von Haar, umfassend eine Begradigungszusammensetzung, die einen pH von 4 oder weniger hat und Glyoxylsäure und/oder ein Hydrat davon und/oder ein Salz davon, und eine Färbezusammensetzung, die zumindest einen Haarfarbstoff, wahlweise zumindest ein Alkalisiermittel und wahlweise zumindest ein Oxidationsmittel enthält.

17. Verwendung des Haarbehandlungskits gemäß Anspruch 16 zum Begradigen und Färben von Haar.

18. Verwendung gemäß Anspruch 17, worin die Begradigung und Färbung entsprechend dem Verfahren gemäß einem der Anspruche 1 bis 15 durchgeführt wird.

## Revendications

1. Procédé pour défriser et teindre les cheveux, **caractérisé en ce qu'**il comprend les étapes suivantes dans cet ordre :
(a) appliquer sur les cheveux une première composition (composition de défrisage) ayant un pH de 4 ou moins et comprenant un acide glyoxylique et/ou un hydrate de celui-ci et/ou un sel de celui-ci ;
(b) laisser la composition sur les cheveux pendant 1 à 120 minutes,
(c) sécher les cheveux
(d) éventuellement rincer les cheveux avec de l'eau et sécher les cheveux,
(e) traiter les cheveux avec un fer ayant une température de surface de 130 à 250°C, de préférence 180 ± 50°C,
(f) éventuellement rincer et/ou faire un shampoing et sécher les cheveux,
(g) appliquer sur les cheveux une seconde composition (composition de teinture) comprenant au moins une teinture capillaire, éventuellement au moins un agent alcalinisant et éventuellement au moins un agent oxydant,
(h) laisser la composition sur les cheveux pendant 5 à 45 minutes, rincer la
composition et éventuellement faire un shampoing et sécher les cheveux, dans lequel les étapes (g) à (h) sont effectuées directement après les étapes (a) à (f).

2. Procédé selon la revendication 1, **caractérisé en ce que** la composition de teinture comprend un ou plusieurs colorants directs, de préférence choisis parmi des colorants anioniques, cationiques et nitro.

3. Procédé selon la revendication 1 ou 2, **caractérisé en ce que** la composition de teinture comprend des précurseurs de colorant oxydatifs et des coupleurs.

4. Procédé selon l'une quelconque des revendications 1 à 3, **caractérisé en ce que** la composition de teinture comprend un agent alcalinisant.

5. Procédé selon l'une quelconque des revendications 1 à 4, **caractérisé en ce que** la composition de teinture comprend un agent oxydant.

6. Procédé selon la revendication 5, **caractérisé en ce que** l'agent oxydant est le peroxyde d'hydrogène.

7. Procédé selon l'une quelconque des revendications 1 à 6, **caractérisé en ce que** la composition de défrisage comprend l'acide glyoxylique et/ou un hydrate de celui-ci et/ou un sel de celui-ci à une concentration dans la plage de 0,1 à 40 % en poids, calculée sur la base du poids total de la composition de défrisage.

8. Procédé selon l'une quelconque des revendications 1 à 7, **caractérisé en ce que** la composition de défrisage a un pH compris entre 0,5 et 3.

9. Procédé selon l'une quelconque des revendications 1 à 8, **caractérisé en ce que** la composition de défrisage et/ou la composition de teinture comprend en outre un ou plusieurs composants de conditionnement.

10. Procédé selon la revendication 9, dans lequel le composant de conditionnement est une silicone.

11. Procédé selon l'une quelconque des revendications 1 à 10, dans lequel la composition de défrisage comprend moins de 2 % d'agents réducteurs à base de soufre.

12. Procédé selon l'une quelconque des revendications 1 à 11, **caractérisé en ce que** la composition de défrisage est exempte de tout agent réducteur à base de soufre.

13. Procédé selon l'une quelconque des revendications 1 à 12, **caractérisé en ce que** la composition de défrisage et/ou la composition de teinture est appliquée sur les cheveux selon un rapport en poids des cheveux à la composition compris entre 0,5:2 et 2:0,5.

14. Procédé selon l'une quelconque des revendications 1 à 13, **caractérisé en ce que** la température du fer est comprise entre 170 et 200 °C.

15. Procédé selon l'une quelconque des revendications 1 à 14, dans lequel la combinaison avec l'application d'une composition réductrice ou d'un agent de relâchement alcalin est exclue.

16. Kit de traitement capillaire pour le défrisage et la teinture des cheveux, comprenant une composition de défrisage, qui a un pH de 4 ou moins et comprend de l'acide glyoxylique et/ou un hydrate de celui-ci et/ou un sel de celui-ci, et une composition de teinture, qui comprend au moins une teinture capillaire, éventuellement au moins un agent alcalinisant et éventuellement au moins un agent oxydant.

17. Utilisation du kit de traitement des cheveux tel que défini dans la revendication 16 pour défriser et teindre les cheveux.

18. Utilisation selon la revendication 17, dans laquelle le défrisage et la teinture sont réalisés selon le procédé tel que défini dans l'une quelconque des revendications 1 à 15.
